# EUROPEAN PATENT APPLICATION

(11) **EP 2 497 466 A2**
(43) Date of publication of application: **12.09.2012**
(21) Application number: 12171186.5
(22) Date of filing: 15.03.2005
(51) Int. Cl.: A61K 9/30, A61K 9/70, A61K 31/426

(54) **Preparation for oral administration**

(30) Priority: 31.03.2004 JP 2004106854
(62) Divisional of application: 05720824.1
(71) Applicant: LINTEC Corporation, Tokyo 173-0001 (JP)
(72) Inventor: Nogami, Eiji, Saitama-shi, Saitama 3360026 (JP)
(74) Representative: Benson, John Everett

(57) **Abstract**

It is an object of the present invention to provide an orally administered pharmaceutical composition capable of completely masking the flavor, odor and the like of a drug contained in a drug-containing layer, and to achieve this object, the outer edge of a first water-swellable gel-forming layer 12a and the outer edge of a second water-swellable gel-forming layer 12' are bonded together so as to enclose a drug-containing layer 11 inside an orally administered pharmaceutical composition 1a in the orally administered pharmaceutical composition 1a comprising the first water-swellable gel-forming layer 12a and the second water-swellable gel-forming layer 12' in the outermost layer.

## Description

### TECHNICAL FIELD

The present invention relates to an orally administered pharmaceutical composition.

### BACKGROUND ART

Compliance may be diminished in the case of an orally administered pharmaceutical composition if it is rejected because the drug is bitter, astringent or otherwise unpleasant or if it causes nausea or vomiting.

For example, a solid preparation (such as a tablet or capsule), which is the normal form of an orally administered pharmaceutical composition, is difficult to swallow as is and must normally be taken with a large amount of water, which may detract from compliance. Elderly patients and infants in particular may be unable to swallow solid preparations, and compliance is often adversely affected. There is also the risk that a solid preparation may lodge in the trachea or adhere to the esophagus, leading to the formation of an esophageal tumor.

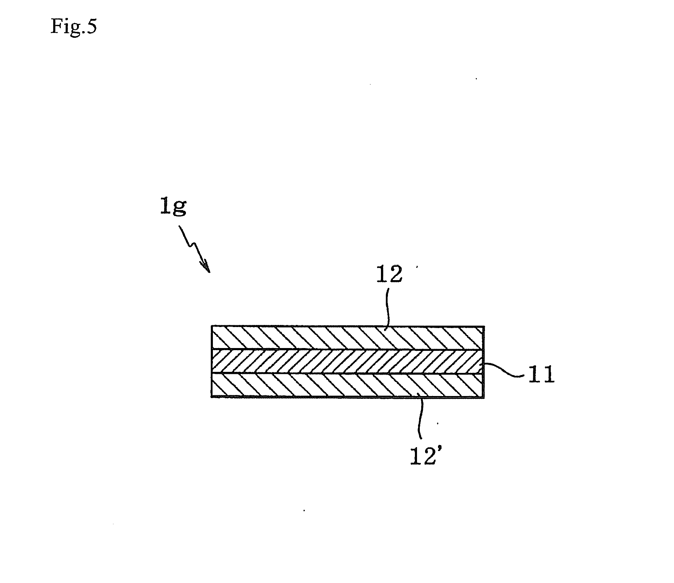

Therefore, as shown in Fig. 5, orally administered pharmaceutical composition 1g comprising water-swellable gel-forming layers 12 and 12' as the outermost layers has been developed by layering water-swellable gel-forming layers 12 and 12' on the top and bottom, respectively, of drug-containing layer 11 (International Patent Publication WO 02/087622).

When orally administered pharmaceutical composition 1g is administered in the mouth of a patient, water-swellable gel-forming layers 12 and 12' swell from saliva or other moisture to form a gel. In this way, orally administered pharmaceutical composition 1g changes to a form of an easily ingestible size, shape, elasticity, viscosity and the like, making it easier to take and reducing the risk of it lodging in the patient's trachea, so that the pharmaceutical composition can be taken safely even by elderly patients and infants. In the case of patients having too little saliva for water-swellable gel-forming layers 12 and 12' to swell adequately and form a gel, the same effects can be obtained by administering the pharmaceutical composition together with a small amount of water or soaking it in water before administration. Much less water is required in this case than is required for administering a tablet, capsule or other solid preparation.

When orally administered pharmaceutical composition 1g is administered in the mouth of a patient, water-swellable gel-forming layers 12 and 12' swell from saliva or other moisture to form a gel, so that drug-containing layer 11 becomes covered in gel. This masks the flavor (such as bitterness or astringency), odor and the like of the drug contained in drug-containing layer 11, so that compliance is not adversely affected.

Moreover, by working orally administered pharmaceutical composition 1g into a film preparation (sheet preparation) it is possible to reduce the moisture content of the preparation below that of a gelatinous preparation containing a large amount of moisture, thereby improving the stability of the drug (particularly in the case of an easily hydrolysable drug), as well as making it easier to handle and reducing packaging costs.

Moreover, since in orally administered pharmaceutical composition 1g drug-containing layer 11 and water-swellable gel-forming layers 12 and 12' are formed independently, even if the film strength of drug-containing layer 11 decreases when the amount of drug in drug containing-layer 11 is increased, the strength of the film preparation as a whole can be maintained by conferring film formability on water-swellable gel-forming layers 12 and 12'. Consequently, drug-containing layer 11 of this orally administered pharmaceutical composition 1g may contain a wide variety of drugs which are administered in tiny to large doses, as well as insoluble and bulky drugs that are likely to detract from film strength.

### DISCLOSURE OF THE INVENTION

However, as shown in Fig. 5, drug-containing layer 11 is exposed in some places at the edges of orally administered pharmaceutical composition 1g. Since these parts do not become covered by gel even when water-swellable gel-forming layers 12 and 12' swell from saliva or other moisture to form a gel, drug-containing layer 11 remains exposed. This means that the flavor, odor and the like of the drug containing in drug-containing layer 11 cannot be completely masked in orally administered pharmaceutical composition 1g.

It is therefore an object of the present invention to provide an orally administered pharmaceutical composition capable of completely masking the flavor, odor and the like of a drug contained in a drug-containing layer.

To solve the aforementioned problem, the present invention provides an orally administered pharmaceutical composition comprising a first water-swellable gel-forming layer and second water-swellable gel forming layer in the outermost layer, wherein the outer edge of the first water-swellable gel-forming layer and the outer edge of the second water-swellable gel-forming layer are bonded together so as to enclose the drug within the orally administered pharmaceutical composition.

In the orally administered pharmaceutical composition of the present invention, the drug contained inside the orally administered pharmaceutical composition is completely covered by the first and second water-swellable gel-forming layers. Consequently, when the orally administered pharmaceutical composition of the present invention is administered in the mouth of a patient, the first and second water-swellable gel-forming layers swell from saliva or other moisture to form a gel, so that the entire drug contained inside the orally administered pharmaceutical composition becomes covered by gel, completely masking the flavor, odor and the like of the drug.

The drug may be enclosed inside the orally administered pharmaceutical composition in any state in the orally administered pharmaceutical composition of the present invention. For example, the drug may be enclosed inside the orally administered pharmaceutical composition in a drug-containing layer or in the form of a tablet, powder, liquid or other appropriate preparation. One mode of the orally administered pharmaceutical composition of the present invention comprises a drug-containing layer provided between the aforementioned first water-swellable gel-forming layer and second water-swellable gel-forming layer, with the outer edges of the first water-swellable gel-forming layer and second water-swellable gel-forming layer bonded together so that the drug-containing layer is completely enclosed inside the orally-administered pharmaceutical composition.

In the orally administered pharmaceutical composition of the present invention, there are no limits on how the outer edge of the first water-swellable gel-forming layer is bonded to the outer edge of the second water-swellable gel-forming layer as long as the drug is enclosed within the orally administered pharmaceutical composition, and the outer edge of the first water-swellable gel-forming layer and the outer edge of the second water-swellable gel-forming layer may be bonded directly or may be bonded via an adhesive layer.

In the orally administered pharmaceutical composition of the present invention, the "outermost layer" is the layer constituting the outer surface of the orally administered pharmaceutical composition when the orally administered pharmaceutical composition is in the mouth of a patient or the like. Consequently, the "outermost layer" may of course be a layer that constitutes the outer surface of the orally administered pharmaceutical composition before administration, or may be a layer that does not constitute the outer surface of the orally administered pharmaceutical composition before administration, but that constitutes the outer surface of the orally administered pharmaceutical composition when it is in the patient's mouth. For example, even if an additional layer is provided as an outer layer outside the water-swellable gel-forming layer, if this outer layer is broken down or dissolved by saliva or other moisture inside the patient's mouth, the water-swellable gel-forming layer will constitute the outer surface of the orally administered pharmaceutical composition inside the patient's mouth, so that the water-swellable gel-forming layer becomes the outermost layer of the orally administered pharmaceutical composition.

An orally administered pharmaceutical composition capable of completely masking the flavor, odor and the like of a drug contained in a drug-containing layer is provided by the present invention.

### BRIEF DESCRIPTION OF THE DRAWINGS

Fig. 1A is a plane view showing the first embodiment of the orally administered pharmaceutical composition of the present invention, and Fig. 1B is a cross-section (X-X cross-section in Fig. 1A) of the same embodiment;
Fig. 2A is a plane view showing the second embodiment of the orally administered pharmaceutical composition of the present invention, and Fig. 2B is a cross-section (X-X cross-section in Fig. 2A) of the same embodiment;
Fig. 3 is a cross-section showing another embodiment of the orally administered pharmaceutical composition of the present invention;
Fig. 4 is a cross-section showing yet another embodiment of the orally administered pharmaceutical composition of the present invention; and
Fig. 5 is a cross-section showing a conventional orally administered pharmaceutical composition.

### BEST MODE FOR CARRYING OUT THE INVENTION

The present invention is explained in detail below based on the drawings.

### [First Embodiment]

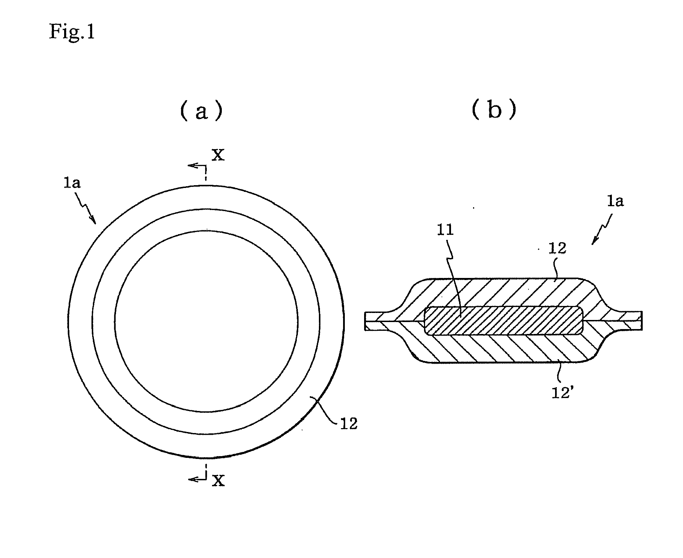

Fig. 1(a) is a plane view showing the first embodiment of the orally administered pharmaceutical composition of the present invention, while Fig. 1(b) is a cross-section (X-X cross-section in Fig. 1(a)) of the same embodiment.

As shown in Fig. 1, orally administered pharmaceutical composition 1a of the first embodiment comprises water-swellable gel-forming layers 12 and 12' in the outermost layer of orally administered pharmaceutical composition 1a and drug-containing layer 11 layered between water-swellable gel-forming layers 12 and 12', with the outer edge of water-swellable gel-forming layer 12 being bonded directly to the outer edge of water-swellable gel-forming layer 12' so that drug-containing layer 11 is enclosed within orally administered pharmaceutical composition 1a.

Orally administered pharmaceutical composition 1a is preferably a film preparation (sheet preparation). When orally administered pharmaceutical composition 1a is a film preparation the moisture content of the preparation can be minimized, thereby making the drug (particularly in the case of an easily hydrolysable drug) contained in drug-containing layer 11 more stable than in a gelatinous preparation containing a large amount of water. The preparation is also easier to handle, and packaging costs can be reduced.

Drug-containing layer 11 is the layer containing the drug to be administered. The drug contained in drug-containing layer 11 is normally contained in drug-containing layer 11 in the form of a suitable preparation, such as a film, powder, tablet or the like. The form of the drug contained in drug-containing layer 11 is not particularly limited as long as it does not detract from formation of drug-containing layer 11. Although drug-containing layer 11 may consist only of the drug to be administered, it normally contains pharmacologically acceptable excipients, binders, disintegrators, masking agents, colorants and the like as bases for maintaining the drug to be administered in the desired state in the drug-containing layer.

The thickness of drug-containing layer 11 can be adjusted appropriately within the range that allows oral administration. When orally administered pharmaceutical composition 1a is a film preparation, the thickness of drug-containing layer 11 is preferably 0.1 to 1000 µm or more preferably 10 to 200 µm. If the thickness of drug-containing layer 11 is under 0.1 µm it will be hard to form the film precisely (that is, the drug content of drug-containing layer 11 will vary), while if the thickness of drug-containing layer 11 is over 1000 µm the film will be stiff and harder to administer.

There are no particular limits on the base contained together with the drug in drug-containing layer 11, which may be selected appropriately according to the object. Specific examples of bases to be contained in drug-containing layer 11 include crystal cellulose, carboxymethyl cellulose, hydroxypropyl cellulose, hydroxypropyl methyl cellulose, methyl cellulose, ethyl cellulose, cellulose acetate, cellulose acetate phthalate, hydroxypropyl methylcellulose phthalate, hydroxypropyl methylcellulose acetate succinate, carboxymethyl ethyl cellulose and other celluloses and derivatives thereof and pharmacologically acceptable salts (such as sodium salts) of these; alpha starch, oxidized starch, carboxymethyl starch sodium, hydroxypropyl starch, dextrin, dextran and other starches and derivatives thereof; sucrose, maltose, lactose, glucose, fructose, pullulan, xanthan gum, cyclodextrin and other sugars; xylitol, mannitol, sorbitol and other sugar alcohols; dimethylaminoethyl methacrylate-methacrylic acid copolymer, methacrylic acid-ethyl acrylate copolymer, methacrylic acid-methyl methacrylate copolymer, ethyl methacrylate-ammonium trimethyl chloride methacrylate copolymer, dimethylaminoethyl methacrylate-methyl methacrylate chloride copolymer, methacrylic acid-ethyl acrylate chloride copolymer and other acrylic acid derivatives; shellac; polyvinyl acetal diethyl aminoacetate; polyvinyl acetate; polyvinyl alcohol; polyvinylpyrrolidone; vinyl acetate-vinylpyrrolidone copolymer; gum arabic, tragacanth and other natural gums; chitin, chitosan and other polyglycosamines; gelatin, casein, soy protein and other proteins; titanium oxide; calcium hydrogenphosphate; calcium carbonate; talc; stearic acid salts; magnesium aluminate metasilicate; magnesium silicate; anhydrous silicic acid and the like, and 1 or 2 or more of these may be selected and used depending on the object.

The base contained in drug-containing layer 11 is preferably an edible polymer. This edible polymer may be either a synthetic polymer or natural polymer, with no particularly limits as to type.

The edible polymer is preferably a gastrosoluble or enterosoluble polymer.

Desirable examples of edible polymers include cellulose and cellulose derivatives, polyvinylpyrrolidone, polyvinyl acetate, vinylpyrrolidone-vinyl acetate copolymer and the like, and particularly desirable examples include hydroxypropyl cellulose, hydroxypropyl methylcellulose phthalate, polyvinylpyrrolidone, polyvinyl acetate and vinylpyrrolidone-vinyl acetate copolymer. Hydroxypropyl cellulose, hydroxypropyl methylcellulose phthalate, polyvinylpyrrolidone, polyvinyl acetate and vinylpyrrolidone-vinyl acetate copolymer have excellent film-forming properties, making them useful when drug-containing layer 11 is in film form.

The amount of the base contained in drug-containing layer 11 is an amount that allows formation of drug-containing layer 11, and can be adjusted appropriately according to the type of base and the like, but is normally at least 20% or preferably at least 60% or more preferably at least 70% by weight of drug-containing layer 11. If the total content of the base is less than 20% by weight drug-containing layer 11 does not form properly. The upper limit on the content of the base is 100% by weight minus the minimum content of the drug contained in drug-containing layer 11, and can be set appropriately according to the type of drug and the like. For example, if the minimum content of the drug is 0.01% by weight of drug-containing layer 11, the upper limit on the content of the base is 99.99% by weight of drug-containing layer 11.

The drug contained in drug-containing layer 11 is a drug to be administered to a patient or the like, and is not particularly limited as long as it is a drug that can be orally administered. Examples of drugs that can be orally administered include drugs that affect the central nervous system, such as amobarbital, estazolam, triazolam, nitrazepam, pentobarbital and other sleeping drugs, amitriptyline hydrochloride, imipramine hydrochloride, oxazolam, chlordiazepoxide, chlorpromazine, diazepam, sulpiride, haloperidol and other psychotropics, trihexyphenidyl, levodopa and other antiparkinsonian drugs, aspirin, isopropylantipyrine, indomethacin, diclofenac sodium, mefenamic acid, streptokinase, streptodornase, serrapeptase, pronase and other analgesics and anti-inflammatories, and ATP, vinpocetine and other central nervous metabolism enhancers; drugs that affect the respiratory system, such as carbocysteine, bromhexine and other expectorants and azelastine hydrochloride, oxatomide, theophylline, terbutaline sulfate, tranilast, procaterol hydrochloride, ketotifen fumarate and other anti-asthmatics; drugs that affect the circulatory system, such as aminophylline, digitoxin, digoxin and other cardiac stimulants, ajmaline, disopyramide, procainamide hydrochloride, mexiletine hydrochloride and other antiarrhythmics, amyl nitrite, alprenolol hydrochloride, isosorbide dinitrate, nicorandil, oxyfedrine, dipyridamole, dilazep hydrochloride, diltiazem hydrochloride, nitroglycerine, nifedipine, verapamil hydrochloride and other anti-angina drugs, kallidinogenase and other peripheral vasodilators, atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine and other anti-hypertensives, and clofibrate, dextran sulfuric acid, nicomol, niceritrol and other anti-arteriosclerotics; blood and hematopoietic drugs, such as carbazochrome sodium sulfate, tranexamic acid and other hemostatics, ticlopidine hydrochloride, warfarin potassium and other anti-thrombosis drugs, and iron sulfate and other anemia treatment drugs; drugs that affect the digestive tract, such as azulene, aldioxa, cimetidine, ranitidine hydrochloride, famotidine, teprenone, rebamipide and other anti-ulcer drugs, domperidone, metoclopramide and other antiemetics, sennoside and other cathartics, digestive enzyme regulators, and glycyrrhizin, liver extract preparations and other drugs for treatment of liver disease; drugs that affect metabolic conditions, such as glibenclamide, chlorpropamide, tolbutamide and other anti-diabetic drugs, and allopurinol, colchicine and other gout treatment drugs; ophthalmological drugs such as acetazolamide; otorhinological drugs, such as diphenidol hydrochloride, betahistine mesylate and other antivertigo drugs; chemotherapy drugs and antibiotics, such as isoniazid, ethambutol hydrochloride, ofloxacin, erythromycin stearate, cefaclor, norfloxacin, fosfomycin calcium, minocycline hydrochloride, rifampicin and rokitamycin; anti-malignant tumor drugs, such as cyclophosphamide and tegafur; immunosuppressors such as azathioprine; hormones and endocrine treatment drugs such as luteal hormone, salivary gland hormone, thiamazole, prednisolone, betamethasone, liothyronine and levothyroxine; autacoids such as clemastine fumarate, D-chlorpheniramine maleate and other antihistamines; and alfacalcidol, cobamamide, tocopherol nicotinate, mecobalamin and other vitamins and the like, and 1 or 2 or more of these can be selected and used according to the object of treatment or prevention or the like.

The amount of the drug contained in drug-containing layer 11 can be adjusted appropriately according to the type of drug and the like, but is normally 80% or less or preferably 40% or less or more preferably 30% or less by weight of drug-containing layer 11. Above 80% by weight of the drug-containing layer, the film strength is reduced when orally administered pharmaceutical composition 1a is a film preparation. The lower limit on the drug content can be set appropriately according to the type of drug contained in drug-containing layer 11, but is normally about 0.01% by weight.

A wide range of drugs that are administered in tiny to large doses may be included in drug-containing layer 11. A tiny dose here means 1 mg or less per administration, while a large dose means 300 mg or more per administration.

When orally administered pharmaceutical composition 1a is a film preparation, drug-containing layer 11 may still contain a wide range of drugs that are administered in tiny to large doses, as well as insoluble and bulky drugs that are likely to detract from film strength. This is because drug-containing layer 11 and water-swellable gel-forming layers 12 and 12' are formed as separate layers, so that even if the film strength of drug-containing layer 11 declines when the amount of drug in drug-containing layer 11 is increased, the strength of the film preparation as a whole is maintained because of the film formability conferred on water-swellable gel-forming layers 12 and 12'.

Water-swellable gel-forming layers 12 and 12' are layers containing a water-swellable gel-forming agent and capable of swelling from moisture to form a gel.

The thickness of water-swellable gel-forming layers 12 and 12' can be set appropriately within a range that allows oral administration, but is preferably 10 to 1000 µm or more preferably 20 to 500 µm when orally administered pharmaceutical composition 1 is a film preparation. If water-swellable gel-forming layers 12 and 12' are less than 10 µm thick the gel will not form properly, and the ability of water-swellable gel-forming layers 12 and 12' to mask the flavor, odor and the like of the drug will be inadequate, while if water-swellable gel-forming layers 12 and 12' are over 1000 µm thick they will not swell adequately to form a gel simply from saliva when administered in a patient's mouth or the like, making the pharmaceutical composition difficult to take.

The water-swellable gel-forming agent contained in water-swellable gel-forming layers 12 and 12' is not particularly limited as to type as long as it can swell from moisture to form a gel, and it may or may not be crosslinked.

Examples of water-swellable gel-forming agents include carboxyvinyl polymers, starch and derivatives thereof, agar, alginic acid, arabinogalactan, galactomannan, cellulose and derivatives thereof, carrageen, dextran, tragacanth, gelatin, pectin, hyaluronic acid, gellan gum, collagen, casein, xanthan gum and the like, and 1 or 2 or more of these can be selected and used.

The water-swellable gel-forming agent contained in water-swellable gel-forming layers 12 and 12' is preferably a crosslinked carboxyvinyl polymer, particularly a crosslinked polyacrylic acid. Crosslinked carboxyvinyl polymers and crosslinked polyacrylic acids in particular do not adversely affect the film-forming capabilities of film-forming agents, and exhibit good gel strength when swollen.

Crosslinking can be accomplished with a crosslinking agent suited to the type of molecule to be crosslinked. A carboxyvinyl polymer can be crosslinked for example with a polyvalent metal compound. Specific examples of such polyvalent metal compounds include calcium chloride, magnesium chloride, aluminum chloride, aluminum sulfate, potassium alum, iron alum chloride, ammonium alum, ferric sulfate, aluminum hydroxide, aluminum silicate, aluminum phosphate, iron citrate, magnesium oxide, calcium oxide, zinc oxide, zinc sulfate and the like, and 1 or 2 or more of these can be selected and used.

The amount of the water-swellable gel-forming agent contained in water-swellable gel-forming layers 12 and 12' can be adjusted appropriately according to the type of water-swellable gel-forming agent and the like, but is preferably 15 to 70% by weight of the water-swellable gel-forming layer.

When orally administered pharmaceutical composition 1a is a film preparation, water-swellable gel-forming layers 12 and 12' need to be made in film form, and in this case it is desirable to include a film-forming agent in water-swellable gel-forming layers 12 and 12' in order to improve the film-forming properties of water-swellable gel-forming layers 12 and 12'.

The film-forming agent is not particularly limited as to type as long as it has film-forming ability. Specific examples of film-forming agents include polyvinyl alcohol, polyvinylpyrrolidone, polyvinyl acetate, polyvinyl acetate phthalate, hydroxyalkyl cellulose (such as hydroxypropyl cellulose, hydroxypropyl methyl cellulose, hydroxymethyl cellulose and hydroxyethyl cellulose), alkyl cellulose (such as methyl cellulose and ethyl cellulose), carboxyalkyl cellulose (such as carboxymethyl cellulose), (meth)acrylic acid and esters thereof, xanthan gum, carrageenan, alginic acid and the like, and 1 or 2 or more of these can be selected and used.

The amount of the film-forming agent contained in water-swellable gel-forming layers 12 and 12' can be adjusted appropriately according to the type of film-forming agent and the like, but is preferably 30 to 85% by weight of water-swellable gel forming layers 12 and 12'.

The film-forming agent contained in water-swellable gel-forming layers 12 and 12' is preferably water-soluble. When the film-forming agent is water-soluble, moisture penetrates water-swellable gel-forming layers 12 and 12' more easily, promoting swelling and gel formation of water-swellable gel-forming layers 12 and 12' in the mouth.

Examples of water-soluble film-forming agents include polyvinyl alcohol, hydroxyalkyl cellulose such as hydroxypropyl cellulose, hydroxypropyl methyl cellulose and methyl cellulose, and polyvinylpyrrolidone, xanthan gum, carrageenan, alginic acid and the like, and 1 or 2 or more of these can be selected and used.

A plasticizer may be included in water-swellable gel-forming layers 12 and 12' in order to confer a suitable degree of flexibility on water-swellable gel-forming layers 12 and 12'. Examples of plasticizers include propylene glycol, polyethylene glycol, glycerin, sorbitol, glycerin triacetate, diethyl phthalate, triethyl citrate, lauric acid, sucrose and the like, and 1 or 2 of these may be selected and used.

A masking agent may be included in water-swellable gel-forming layers 12 and 12' in order to mask the flavor, odor and the like of the drug contained in drug-containing layer 11. Including a masking agent in water-swellable gel-forming layers 12 and 12' serves to improve the ability of water-swellable gel-forming layers 12 and 12' to mask the flavor, odor and the like of the drug. Examples of masking agents include those that contribute acidity such as citric acid, tartaric acid and fumaric acid, sweeteners such as saccharine, glycyrrhizic acid, sucrose, fructose and mannitol, cooling agents such as menthol, peppermint and spearmint, and natural and artificial aromatics and the like, and 1 or 2 or more of these can be selected and used.

When polyvinyl alcohol and the like are included as film-forming agents in water-swellable gel-forming layers 12 and 12', these film-forming agents may also serve as masking agents. It is desirable to use a film-forming agent having such a masking effect, and it is similarly desirable to use a water-swellable gel-forming agent having such a masking effect.

Preservatives such as methyl- and propyl-hydroxybenzoate and colorants such as edible lake colorants can also be included in water-swellable gel-forming layers 12 and 12'.

In general, mixing of these additives into water-swellable gel-forming layers 12 and 12' weakens water-swellable gel-forming layers 12 and 12', making it easier for moisture to penetrate these layers, so that the water-swellable gel-forming agent swells and forms a gel more easily due to moisture penetrating water-swellable gel-forming layers 12 and 12'.

As shown in Fig. 1, in orally administered pharmaceutical composition 1a the top and bottom surfaces of drug-containing layer 11 are covered, respectively, by the centers (parts surrounded by outer edges) of water-swellable gel-forming layers 12 and 12', while the sides of drug-containing layer 11 are covered by the bonded outer edges of water-swellable gel-forming layers 12 and 12'. That is, all of drug-containing layer 11 is covered by water-swellable gel-forming layers 12 and 12'. Consequently, when orally administered pharmaceutical composition 1a is administered in the mouth of a patient, water-swellable gel-forming layers 12 and 12' swell from saliva or other moisture to form a gel, and drug-containing layer 11 becomes entirely covered by gel, completely masking the flavor, odor and the like of the drug contained in drug-containing layer 11.

As shown in Fig. 1, water-swellable gel-forming layers 12 and 12' are provided in the outermost layer of orally administered pharmaceutical composition 1a. Consequently, when water-swellable gel-forming layers 12 and 12' gel, orally administered pharmaceutical composition 1a changes to a form of an easy-to-swallow size, shape, elasticity, viscosity and the like. In this way, a patient can easily take orally administered pharmaceutical composition 1a. Since there is also less risk that orally administered pharmaceutical composition 1a will lodge in the patient's trachea during administration, it can be given safely even to elderly patients and infants. In the case of patients who do not have enough saliva to adequately gel water-swellable gel-forming layers 12 and 12', the same effects can be obtained by administering the pharmaceutical composition together with a small amount of water or by soaking it in water before administration. Much less water is required in this case than is required for administering a tablet, capsule or other solid preparation.

Orally administered pharmaceutical composition 1a can be produced for example by the following methods.

### [First Production Method]

A suspension containing a water-swellable gel-forming agent and a film-forming agent (with purified water for example as the solvent) is painted, sprayed or otherwise applied to the upper surface of a plastic film, mount or other support base, and dried to form water-swellable gel-forming layer 12, thereby producing a first layered body comprising water-swellable gel-forming layer 12 layered on the upper surface of a support base.

Water-swellable gel-forming layer 12' is formed in the same way on the upper surface of a plastic film, mount or other support base and a suspension containing a drug and excipients, binders, disintegrators and other additives (with ethanol for example as the solvent) is painted, sprayed or otherwise applied to the upper surface of water-swellable gel-forming layer 12' and dried to form drug-containing layer 11. In this case, the size of the bottom surface of drug-containing layer 11 is made smaller than the size of the top surface of water-swellable gel-forming layer 12' (that is, drug-containing layer 11 is formed in the center of the upper surface of water-swellable gel-forming layer 12' so that the outer edges of the upper surface of water-swellable gel-forming layer 12' remain exposed). The method of forming drug-containing layer 11 is not limited to the aforementioned methods, and for example drug-containing layer 11 can be formed on the upper surface of water-swellable gel-forming layer 12' by printing using a known method such as screen printing. In this way, a second layered body is produced comprising water-swellable gel-forming layer 12' and drug-containing layer 11 layered successively on a support base.

Next, the surface of the outer edge of water-swellable gel-forming layer 12 and the surface of the outer edge of water-swellable gel-forming layer 12' are moistened with water to gel them, and the gelled outer edges are pressed together and then dried. In this case, the parts in contact gel and dry as a unit, so that the outer edge of water-swellable gel-forming layer 12 and the outer edge of water-swellable gel-forming layer 12' bond directly to one another. By directly bonding the edge of water-swellable gel-forming layer 12 with the outer edge of water-swellable gel-forming layer 12' in this way, it is possible to produce orally administered pharmaceutical composition 1a comprising drug-containing layer 11 enclosed on the inside.

### [Second Production Method]

A first layered body comprising water-swellable gel-forming layer 12 layered on the top surface of a support base and a second layered body comprising water-swellable gel-forming layer 12' layered on the top surface of a support base are produced as in the first production method.

Meanwhile, a suspension containing a drug and excipients, binders, disintegrators and other additives (with ethanol for example as the solvent) is painted, sprayed or otherwise applied to the top surface of a plastic film, mount or other support base, and dried to form a drug-containing film. The drug-containing film thus formed is peeled off the support base, and the resulting drug-containing film is set on the upper surface of water-swellable gel-forming layer 12 of the first layered body or water-swellable gel-forming layer 12' of the second layered body.

Next, the outer edge of water-swellable gel-forming layer 12 of the first layered body and the outer edge of water-swellable gel-forming layer 12' of the second layered body can be bonded directly to one another to produce orally administered pharmaceutical composition 1a comprising a drug-containing film enclosed on the inside.

An orally administered pharmaceutical composition 1a comprising water-swellable gel-forming layer 12', drug-containing layer 11 and water-swellable gel-forming layer 12 layered in that order with drug-containing layer 11 enclosed on the inside can be produced by the aforementioned first or second production method. Orally administered pharmaceutical composition 1a can be punched out in a circular, oval, polygonal or any other shape as necessary. When punching out orally administered pharmaceutical composition 1a, the part where the outer edge of water-swellable gel-forming layer 12 of the first layered body is bonded to the outer edge of water-swellable gel-forming layer 12' of the second layered body is punched so as not to expose drug-containing layer 11.

### [Second Embodiment]

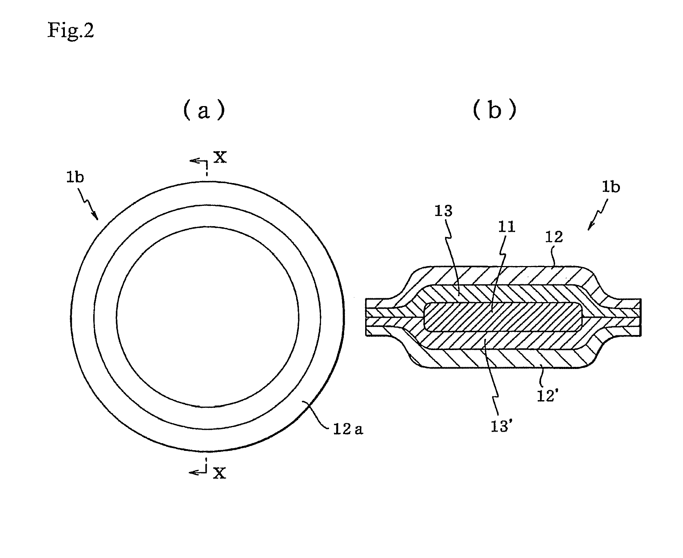

Fig. 2A is a plane view showing the second embodiment of the orally administered pharmaceutical, composition of the present invention, while Fig. 2B is a cross-section (X-X cross-section in Fig. 2A) showing the same embodiment.

As shown in Fig. 2, orally administered pharmaceutical composition 1b of the second embodiment comprises water-swellable gel-forming layers 12 and 12' in the outer layer of orally administered pharmaceutical composition 1b, adhesive layer 13 layered on the bottom surface of water-swellable gel-forming layer 12, adhesive layer 13' layered on the top surface of water-swellable gel-forming layer 12', and drug-containing layer 11 layered between water-swellable gel-forming layers 12 and 12' via adhesive layers 13 and 13', with the outer edge of water-swellable gel-forming layer 12 bonded to the outer edge of water-swellable gel-forming layer 12' by means of adhesive layers 13 and 13' so that drug-containing layer 11 is enclosed within orally administered pharmaceutical composition 1. In Fig. 2, the parts that are the same as in Fig. 1 are labeled with the same symbols, and those explanations that are not particularly necessary are omitted.

Orally administered pharmaceutical composition 1b differs from orally administered pharmaceutical composition 1a in terms of the mode of adhesion between the outer edge of water-swellable gel-forming layer 12 and the outer edge of water-swellable gel-forming layer 12', but as in orally administered pharmaceutical composition 1a, drug-containing layer 11 is entirely covered by water-swellable gel-forming layers 12 and 12'. Moreover, water-swellable gel-forming layers 12 and 12' are in the outermost layer as in orally administered pharmaceutical composition 1a. Consequently, the same effects are provided by orally administered pharmaceutical composition 1b as by orally administered pharmaceutical composition 1a.

The adhesive contained in adhesive layers 13 and 13' is not particularly limited as long as it is a pharmacologically acceptable adhesive. Examples of adhesives that exhibit adhesiveness when included in a solvent include carboxyvinyl polymers, sodium polyacrylate and other polyacrylic acids or pharmacologically acceptable non-toxic salts thereof, acrylic acid copolymers or pharmacologically acceptable salts thereof, carboxymethylcellulose, sodium salts and other hydrophilic cellulose derivatives, pullulan, povidone, karaya gum, pectin, xanthan gum, tragacanth, alginic acid, gum arabic, acidic polysaccharides and derivatives and pharmacologically acceptable salts thereof and the like, and 1 or 2 or more of these may be selected and used. Examples of adhesives that exhibit adhesiveness when heated (that is, heat-fusable adhesives) include for example vinyl acetate, polyvinylpyrrolidone and other homopolymers and copolymers of vinyl acetate and vinylpyrrolidone and the like, and 1 or 2 or more of these may be selected and used.

The thickness of adhesive layers 13 and 13' can be adjusted appropriately within a range that allows oral administration, but is preferably 1 to 50 µm or more preferably 10 to 30 µm when orally administered pharmaceutical composition 1b is a film preparation. If adhesive layers 13 and 13' are less than 1 µm thick they may not adhere properly, while if adhesive layers 13 and 13' are more than 50 µm thick they may impede swelling of orally administered adhesive 1b from saliva and the like during administration, and may also make taking the drug unpleasant if the adhesive contained in adhesive layers 13 and 13' is insoluble in water.

Orally administered pharmaceutical composition 1b may be produced for example by the following methods.

### [First Production Method]

A suspension containing a water-swellable gel-forming agent and a film-forming agent (with purified water for example as the solvent) is painted, sprayed or otherwise applied to the upper surface of a plastic film, mount or other support base, and dried to form water-swellable gel-forming layer 12. Next, a suspension containing an adhesive (with ethanol for example as the solvent) is painted, sprayed or otherwise applied to the upper surface of water-swellable gel-forming layer 12, and dried to form adhesive layer 13. A first layered body comprising water-swellable gel-forming layer 12 and adhesive layer 13 layered in that order on a support base is produced in this way.

Water-swellable gel-forming layer 12' and adhesive layer 13' are formed successively in the same way. Next, a suspension containing a drug and excipients, binders, disintegrators and other additives (with ethanol for example as the solvent) is painted, sprayed or otherwise applied to the upper surface of adhesive layer 1-3', and dried to form drug-containing layer 11. In this case, the size of the lower surface of drug-containing layer 11 is made smaller than the size of the upper surface of adhesive layer 13' (that is, drug-containing layer 11 is formed in the center of the upper surface of adhesive layer 13' so that the outer edges of the upper surface of adhesive layer 13' remain exposed). The method of, forming drug-containing layer 11 is not confined to the aforementioned method, and for example drug-containing layer 11 can also be formed on the upper surface of adhesive layer 13' by printing using a known method such as screen printing or the like. A second layered body comprising water-swellable gel-forming layer 12', adhesive layer 13' and drug-containing layer 11 layered successively on a support base is produced in this way.

Next, the outer edge of water-swellable gel-forming layer 12 of the first layered body and the outer edge of water-swellable gel-forming layer 12' of the second layered body are bonded together via adhesive layers 13 and 13' to produce orally administered pharmaceutical composition 1b comprising drug-containing layer 11 enclosed on the inside. In this case, the desired mode of adhesion can be selected by selecting the adhesive contained in adhesive layers 13 and 13'. When adhesive layers 13 and 13' contain a heat-fusable adhesive, they can be bonded by heat fusion. Heat fusion can be performed at a temperature of normally 60 to 150°C or preferably 90 to 120°C under normally at least 0.1 kgf/cm² preferably at least 0.5 kgf/cm² for normally 0.1 to 5 seconds or preferably 0.5 to 3 seconds.

### [Second Production Method]

A first layered body comprising water-swellable gel-forming layer 12 and adhesive layer 13 layered successively on a support base and a second layered body comprising water-swellable gel-forming layer 12' and adhesive layer 13' layered successively on a support base are produced as in first production method.

Meanwhile, a suspension containing a drug and excipients, binders, disintegrators and other additives (with ethanol for example as the solvent) is painted, sprayed or otherwise applied to the upper surface of a plastic film, mount or other support member, and dried to form drug-containing layer 11. The resulting drug-containing layer 11 is peeled from the support member to obtain a drug-containing film which is the set on adhesive layer 13 of the first layered body or adhesive layer 13' of the second layered body.

Next, the outer edge of adhesive layer 13 of the first layered body can be bonded to the outer edge of adhesive layer 13' of the second layered body as described above to produce orally administered pharmaceutical composition 1b comprising a drug-containing film enclosed on the inside.

Orally administered pharmaceutical composition 1b comprising water-swellable gel-forming layer 12', adhesive layer 13', drug-containing layer 11, adhesive layer 13 and water-swellable gel-forming layer 12 layered in that order with drug-containing layer 11 enclosed on the inside is produced by the aforementioned first or second production method. Orally administered pharmaceutical composition 1b may also be punched out in a round, oval or polygonal shape or in any other shape as necessary. When punching out orally administered pharmaceutical composition 1b, the part where the outer edge of water-swellable gel-forming layer 12 of the first layered body is bonded to the outer edge of water-swellable gel-forming layer 12' of the second layered body is punched so as not to expose drug-containing layer 11.

The following alterations are possible in orally administered pharmaceutical compositions 1a and 1b.

Orally administered pharmaceutical compositions 1a and 1b may have functional layers other than water-swellable gel-forming layers and adhesive layers. An example of such a functional layer is a layer for purposes of adjusting film thickness. When orally administered pharmaceutical compositions 1a and 1b are film preparations, orally administered pharmaceutical compositions 1a and 1b can be made easier to handle by using such a layer to increase the film thickness. Such a functional layer is provided between water-swellable gel-forming layers 12 and 12'.

Orally administered pharmaceutical compositions 1a and 1b each have 1 drug-containing layer, but the number of drug-containing layers is not particularly limited, and orally administered pharmaceutical compositions 1a and 1b may have multiple drug-containing layers. When orally administered pharmaceutical compositions 1a and 1b have multiple drug-containing layers, the drug-containing layers may be layered directly or via an intermediate layer. Moreover, one drug-containing layer may be constituted by multiple drug-containing layers formed side by side.

Orally administered pharmaceutical compositions 1a and 1b each have 2 water-swellable gel-forming layers, but they may also have another water-swellable gel-forming layer. Such a water-swellable gel-forming layer may be provided between water-swellable gel-forming layer 12 and water-swellable gel-forming layer 12', or may be provided outside water-swellable gel-forming layers 12 and 12'.

A drug-containing layer is enclosed inside orally administered pharmaceutical compositions 1a and 1b, but a drug that does not form a drug-containing layer could also be enclosed. For example, a drug formulated in a tablet, powder or other appropriate form could be enclosed without forming a drug-containing layer. A drug can be easily enclosed inside orally administered pharmaceutical compositions 1a and 1b using a formulated drug (see second production method above). By enclosing an already formulated drug in orally administered pharmaceutical compositions 1a and 1b it is possible to limit the amount of excess drug used during the production of orally administered pharmaceutical compositions 1a and 1b, thereby reducing costs.

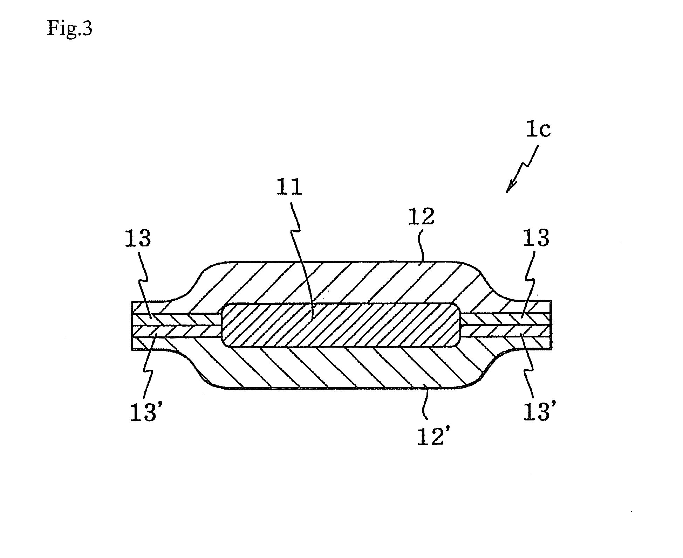

In orally administered pharmaceutical composition 1b adhesive layers 13 and 13' are layered over the entire lower surface of water-swellable gel-forming layer 12 and the entire upper surface of water-swellable gel-forming layer 12', respectively, but the sizes and positions of adhesive layers 13 and 13' are not particularly limited as long as they allow the outer edge of water-swellable gel-forming layer 12 to be bonded to the outer edge of water-swellable gel-forming layer 12'. For example, adhesive layers 13 and 13' may be layered on one part (the outer edge) of the lower surface of water-swellable gel-forming layer 12 and one part (the outer edge) of the upper surface of water-swellable gel-forming layer 12', respectively, as in orally administered pharmaceutical composition 1c shown in Fig. 3.

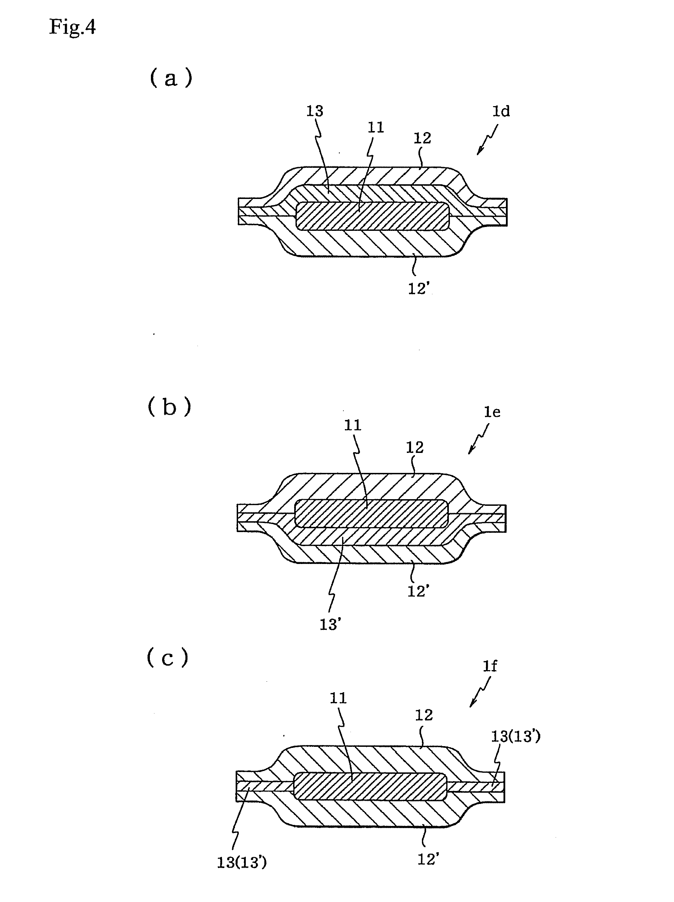

Orally administered pharmaceutical composition 1b comprises 2 adhesive layers 13 and 13' between the outer edge of water-swellable gel-forming layer 12 and the outer edge of water-swellable gel-forming layer 12', but the number of adhesive layers provided between the outer edge of water-swellable gel-forming layer 12 and the outer edge of water-swellable gel-forming layer 12' is not particularly limited as long as it allows the outer edge of water-swellable gel-forming layer 12 to be bonded to the outer edge of water-swellable gel-forming layer 12'. For example, there may be only 1 adhesive layer between the outer edge of water-swellable gel-forming layer 12 and the outer edge of water-swellable gel-forming layer 12' as in orally administered pharmaceutical compositions 1d through 1f shown in Figs. 4A through 4C.

### [Examples]

The present invention is explained in more detail below by means of production examples and test examples.

### (1) Preparation of water-swellable gel-forming layer forming liquid (Coating Liquid A)

Coating liquid A was prepared with the following composition for purposes of forming the water-swellable gel-forming layers. 1 g of potassium alum was added to 140 g of purified water, and completely dissolved by agitation for about 10 minutes. Next, 6 g of polyacrylic acid (Carbopol 974P. BF Goodrich) was added gradually with agitation, and completely dissolved by being agitated for about 1 hour. Next, 17 g of polyvinyl alcohol (Gohsenol EG-05T, Nippon Gohsei) was added gradually with agitation, and completely dissolved by agitation with heating at 70°C for about 1 hour.

Polyacrylic acid is crosslinked by the aluminum ions produced by ionization of potassium alum, and the crosslinked polyacrylic acid serves as a water-swellable gel-forming agent, while the polyvinyl alcohol serves as a film-forming agent.

### (2) Preparation of adhesive layer forming liquid (Coating Liquid B)

Coating Liquid B was prepared with the following composition for purposes of forming the adhesive layer. That is, 6 g of polyvinylpyrrolidone (PVP K-90, ISP Japan) was added gradually with agitation to 25 g of ethanol, and 1 g of glycerin was then added and completely dissolved by agitation for about 20 minutes. Polyvinylpyrrolidone is heat-fusable because it is a thermoplastic polymer.

### (3) Preparation of drug-containing layer forming liquid (Coating Liquid C)

Coating liquid C was prepared with the following composition for purposes of forming the drug-containing layer. That is, 7 g of the stomach ulcer drug famotidine and 0.2 g of titanium oxide were added to ethanol or purified water and thoroughly dispersed with a homogenizer, after which 20 g of any of the bases (binders) listed under (a) through (k) below was added and completely dissolved by being agitated for about 20 minutes:
(a) Polyvinylpyrrolidone (PVP K-30, ISP Japan)
(b) Polyvinylpyrrolidone-vinyl acetate copolymer (S-630, ISP Japan)
(c) Carboxymethyl cellulose sodium (Kanto Chemical Co., Inc.)
(d) Hydroxypropyl cellulose (HPC SL Grade, Nippon Soda)
(e) Gum arabic
(f) Guar gum
(g) Xanthan gum
(h) Gum tragacanth
(i) Locust bean gum
(j) Carageenan
(k) Sodium alginate.

The amount of the solvent was adjusted appropriately so as to achieve a viscosity of 2000 to 6000 mPa•s.

### [Production Example 1] Production of orally administered pharmaceutical composition A

### (1) Formation of water-swellable gel-forming layer

Coating Liquid A was thoroughly degassed and, using an applicator with the gap adjusted so as to achieve a thickness of 30 µm after drying, spread on the opposite side of a polyethylene terephthalate film (Lintec Corporation, SP-PET3801) which had been release-treated on one side with a silicone resin, and dried for 10 minutes at 80°C to form a water-swellable gel-forming layer. Layered body A was thus produced comprising a water-swellable gel-forming layer layered on the aforementioned polyethylene terephthalate film. Another layered body A was produced in the same way.

### (2) Formation of drug-containing layer

Coating Liquid C was thoroughly degassed and 100 µL was dripped onto the top surface of the water-swellable gel-forming layer of a layered body A and dried for about 15 minutes at 80°C to form a drug-containing layer. The drug-containing layer in this case was formed not on the entire top surface of the water-swellable gel-forming layer but only on part of the top surface. That is, the drug-containing layer was layered on the center (area about 1.8 cm²) of the top surface (area about 4.9 cm²) of the water-swellable gel-forming layer so as not to cover the outer edges of the water-swellable gel-forming layer. In this way, a layered body B was produced comprising a water-swellable gel-forming layer and drug-containing layer layered successively on the aforementioned polyethylene terephthalate film.

### (3) Production of orally administered pharmaceutical composition by direct adhesion of water-swellable gel-forming layers

Purified water was applied to gel the surface of the outer edge of the water-swellable gel-forming layer of layered body A and the surface of the outer edge of the water-swellable gel-forming layer of layered body B, the gelled outer edges were pressed together, one of the polyethylene terephthalate films was peeled off, and the whole was dried for about 5 minutes at 80°C to directly bond the outer edge of the water-swellable gel-forming layer of layered body A with the outer edge of the water-swellable gel-forming layer of layered body B. In this way, a layered body was produced comprising a water-swellable gel-forming layer, drug-containing layer and water-swellable gel-forming layer layered successively on the aforementioned polyethylene terephthalate film with the drug-containing layer enclosed on the inside, and this layered body was punched out to produce orally administered pharmaceutical composition A. When punching out the layered body, the part where the water-swellable gel-forming layers were bonded to one another was punched so as not to expose the drug-containing layer.

### [Production Example 2] Production of orally administered pharmaceutical composition B

### (1) Formation of water-swellable gel-forming layer

Coating Liquid A was thoroughly degassed and, using an applicator with the gap adjusted so as to obtain a dried thickness of 30 µm, spread on the opposite side of a polyethylene terephthalate film (Lintec Corporation, SP-PET3801) which had been release-treated on one side with a silicone resin, and dried for 10 minutes at 80°C to form a water-swellable gel-forming layer.

### (2) Formation of adhesive layer

Coating Liquid B was thoroughly degassed and, using an applicator with the gap adjusted so as to obtain a dried thickness of 20 µm, spread on the entire top surface of the aforementioned water-swellable gel-forming layer, and dried for about 3 minutes at 80°C to form an adhesive layer. In this way, layered body C was produced comprising a water-swellable gel-forming layer and adhesive layer layered in that order on the aforementioned polyethylene terephthalate film. Another layered body C was produced in the same way.

### (3) Formation of drug-containing layer

Coating Liquid C was thoroughly degassed, and 100 µL was dripped onto the top surface of the adhesive layer of layered body C and dried for about 15 minutes at 80°C to form a drug-containing layer. In this case, the drug-containing layer was formed on only part of the top surface, not on the entire top surface of the adhesive layer. That is, the drug-containing layer was formed in the center (area about 1.8 cm²) of the top surface (area about 4.9 cm²) of the adhesive layer so as not to cover the outer edges of the adhesive layer. In this way, a layered body D was produced comprising a water-swellable gel-forming layer, an adhesive layer and a drug-containing layer layered in that order on the aforementioned polyethylene terephthalate film.

### (4) Production of orally administered pharmaceutical composition by heat fusion of adhesive layers

The outer edge of the adhesive layer of layered body C and the outer edge of the adhesive layer of layered body D were heat fused together under conditions of 100°C, 1 kgf/cm², 2 seconds. In this way, a layered body was produced comprising a water-swellable gel-forming layer, adhesive layer, drug-containing layer, adhesive layer and water-swellable gel-forming layer layered in that order on the aforementioned polyethylene terephthalate film, and this layered body was punched out to produce orally administered pharmaceutical composition B. When punching out this layered body, the part where the adhesive layers had been heat fused to each other was punched out so as not to expose the drug-containing layer.

### [Production Method 3] Production of orally administered pharmaceutical compositions C through F

### (1) Production of orally administered pharmaceutical composition C

7 g of the stomach ulcer drug famotidine and 0.2 g of titanium oxide were added to ethanol and thoroughly dispersed with a homogenizer, after which 20 g of polyvinylpyrrolidone (PVP K-90, ISP Japan) was added gradually with agitation and thoroughly dissolved by being agitated for about 20 minutes. The amount of solvent was adjusted appropriately so as to achieve a viscosity of 2000 to 6000 mPa•s.

The resulting coating liquid was thoroughly degassed and, using an applicator with the gap adjusted so as to obtain a dried thickness of 70 µm, spread on the opposite side of a polyethylene terephthalate film (Lintec Corporation, SP-PET3801) which had been release treated on one side with silicone resin, and dried for about 15 minutes at 80°C to form the drug-containing layer. Next, the drug-containing layer was peeled of the polyethylene terephthalate film to obtain a drug-containing film.

This drug-containing film was set on the adhesive layer of layered body C, and the outer edge of the adhesive layer of this layered body C and the outer edge of the adhesive layer of another layered body C were heat fused together under conditions of 100°C, 1 kgf/cm², 2 seconds. The drug-containing film was set in the middle (area about 1.8 cm²) of the top surface (area about 4.9 cm²) of the adhesive layer. In this way, a layered body was produced comprising a water-swellable gel-forming layer, adhesive layer, drug-containing film, adhesive layer and water-swellable gel-forming layer layered in that order on the aforementioned polyethylene terephthalate film, with the drug-containing film enclosed inside the layered body, and this layered body was punched out to produce orally administered pharmaceutical composition C. When punching out the layered body, the part where the adhesive layers had been heat-fused together was punched out so as not to expose the drug-containing film.

### (2) Production of orally administered pharmaceutical composition D

Using a 60 mesh (140 µm wire) screen with a 15 mm φ block formed thereon, the coating liquid prepared in (1) above was screen printed on the center (area about 1.8 cm²) of the top surface (area about 4.9 cm²) of the adhesive layer of a layered body C, and dried for about 15 minutes at 80°C. This was repeated until the dried thickness was 70 µm to form a drug-containing layer. In this way, layered body E was produced comprising a water-swellable gel-forming layer, adhesive layer and drug-containing layer layered successively on the aforementioned polyethylene terephthalate film. The outer edge of the adhesive layer of a layered body C and the outer edge of the adhesive layer of layered body E were heat fused together under conditions of 100°C, 1 kgf/cm², 2 seconds. In this way, a layered body was produced comprising a water-swellable gel-forming layer, an adhesive layer, a drug-containing layer, an adhesive layer and a water-swellable gel-forming layer layered in that order on the aforementioned polyethylene terephthalate film with the drug-containing layer enclosed on the inside, and this layered body was punched out to produce orally administered pharmaceutical composition D. When punching out the layered body, the part where the adhesive layers had been heat-fused together was punched out so as not to expose the drug-containing film.

### (3) Production of orally administered pharmaceutical composition E

500 mg of powder obtained by thoroughly mixing famotidine and lactose (excipient) in proportions of 1:49 by weight in a mortar was sprinkled on the top surface of the adhesive layer of layered body C, and the outer edge of the adhesive layer of this layered body C was heat fused with the outer edge of the adhesive layer of another layered body C under conditions of 100°C, 1 kgf/cm², 2 seconds. In this way, a layered body was produced comprising a water-swellable gel-forming layer, an adhesive layer, a drug powder, an adhesive layer and a water-swellable gel-forming layer layered in that order on the aforementioned polyethylene terephthalate film with the drug powder enclosed on the inside, and this layered body was punched out to produce orally administered pharmaceutical composition E. When punching out the layered body, the part where the adhesive layers had been heat-fused together was punched out so as not to expose the drug powder.

### (4) Production of orally administered pharmaceutical composition F

A mixed powder of famotidine and polyvinylpyrrolidone (PVP K-90, ISP Japan) in proportions of 1:49 by weight was tablet molded with a tableting machine using the KBr method. The resulting tablet was set on the upper surface of the adhesive layer of a layered body C, and the outer edge of the adhesive layer of this layered body C was heat fused with the outer edge of the adhesive layer of another layered body C under conditions of 100°C, 1 kgf/cm², 2 seconds. In this way, a layered body was produced comprising a water-swellable gel-forming layer, an adhesive layer, a tablet, an adhesive layer and a water-swellable gel-forming layer layered in that order on the aforementioned polyethylene terephthalate film, and this layered body was punched out to produce orally administered pharmaceutical composition F. When punching out the layered body, the part where the adhesive layers had been heat-fused together was punched out so as not to expose the tablet.

### [Test Example 1] Test to evaluate masking of drug flavor

The orally administered pharmaceutical composition A produced in Production Example 1 and the orally administered pharmaceutical composition B produced in Production Example 2 were given with water to 10 randomly selected test subjects, and the ability to mask the flavor of the drug was evaluated according to the following 3-point scale. The results for orally administered pharmaceutical composition A are shown in Table 1, and the results for orally administered pharmaceutical composition B in Table 2.

### [Evaluation of masking]

- 1: Drug flavor detected
- 2: Slight drug flavor detected
- 3: No drug flavor detected

**[Table 1]**

| Type of base in drug-containing layer | Test subject | | | | | | | | | | Mean |
|---|---|---|---|---|---|---|---|---|---|---|---|
| | 1 | 2 | 3 | 4 | 5 | 6 | 7 | 8 | 9 | 10 | |
| a | 3 | 3 | 3 | 3 | 3 | 3 | 3 | 3 | 3 | 3 | 3 |
| b | 3 | 3 | 3 | 3 | 3 | 3 | 3 | 3 | 3 | 3 | 3 |
| c | 3 | 3 | 3 | 3 | 3 | 3 | 3 | 3 | 3 | 3 | 3 |
| d | 3 | 3 | 3 | 3 | 3 | 3 | 3 | 3 | 3 | 3 | 3 |
| e | 3 | 3 | 3 | 3 | 3 | 3 | 3 | 3 | 3 | 3 | 3 |
| f | 3 | 3 | 3 | 3 | 3 | 3 | 3 | 3 | 3 | 3 | 3 |
| g | 3 | 3 | 3 | 3 | 3 | 3 | 3 | 3 | 3 | 3 | 3 |
| h | 3 | 3 | 3 | 3 | 3 | 3 | 3 | 3 | 3 | 3 | 3 |
| i | 3 | 3 | 3 | 3 | 3 | 3 | 3 | 3 | 3 | 3 | 3 |
| j | 3 | 3 | 3 | 3 | 3 | 3 | 3 | 3 | 3 | 3 | 3 |
| k | 3 | 3 | 3 | 3 | 3 | 3 | 3 | 3 | 3 | 3 | 3 |

**[Table 2]**

| Type of base in drug-containing layer | Test subject | | | | | | | | | | Mean |
|---|---|---|---|---|---|---|---|---|---|---|---|
| | 1 | 2 | 3 | 4 | 5 | 6 | 7 | 8 | 9 | 10 | |
| a | 3 | 3 | 3 | 3 | 3 | 3 | 3 | 3 | 3 | 3 | 3 |
| b | 3 | 3 | 3 | 3 | 3 | 3 | 3 | 3 | 3 | 3 | 3 |
| c | 3 | 3 | 3 | 3 | 3 | 3 | 3 | 3 | 3 | 3 | 3 |
| d | 3 | 3 | 3 | 3 | 3 | 3 | 3 | 3 | 3 | 3 | 3 |
| e | 3 | 3 | 3 | 3 | 3 | 3 | 3 | 3 | 3 | 3 | 3 |
| f | 3 | 3 | 3 | 3 | 3 | 3 | 3 | 3 | 3 | 3 | 3 |
| g | 3 | 3 | 3 | 3 | 3 | 3 | 3 | 3 | 3 | 3 | 3 |
| h | 3 | 3 | 3 | 3 | 3 | 3 | 3 | 3 | 3 | 3 | 3 |
| i | 3 | 3 | 3 | 3 | 3 | 3 | 3 | 3 | 3 | 3 | 3 |
| j | 3 | 3 | 3 | 3 | 3 | 3 | 3 | 3 | 3 | 3 | 3 |
| k | 3 | 3 | 3 | 3 | 3 | 3 | 3 | 3 | 3 | 3 | 3 |

As shown in Tables 1 and 2, the flavor of the drug contained in the drug-containing layer was completely masked regardless of the type of base contained in the drug-containing layer whether the outer edges of the water-swellable gel-forming layers were bonded directly to each other (orally administered pharmaceutical composition A) or were bonded to each other via adhesive layers (orally administered pharmaceutical composition B).

### [Test Example 2]

The ability to mask the flavor of the drug was evaluated as in Test Example 1 with respect to the orally administered pharmaceutical compositions C through F produced in Production Example 3. The results are shown in Table 3.

**[Table 3]**

| Type of orally administered pharmaceutical composition | Test subject | | | | | | | | | | Mean |
|---|---|---|---|---|---|---|---|---|---|---|---|
| | 1 | 2 | 3 | 4 | 5 | 6 | 7 | 8 | 9 | 10 | |
| C | 3 | 3 | 3 | 3 | 3 | 3 | 3 | 3 | 3 | 3 | 3 |
| D | 3 | 3 | 3 | 3 | 3 | 3 | 3 | 3 | 3 | 3 | 3 |
| E | 3 | 3 | 3 | 2 | 3 | 3 | 3 | 3 | 3 | 3 | 2.9 |
| F | 3 | 3 | 3 | 3 | 3 | 2 | 3 | 2 | 3 | 3 | 2.8 |

As shown in Table 3, the flavor of the drug contained in the drug-containing layer was entirely masked regardless of the form of the drug enclosed inside the orally administered pharmaceutical composition.

### INDUSTRIAL APPLICABILITY

An orally administered pharmaceutical composition capable of completely masking the flavor, odor and the like of a drug contained in a drug-containing layer is provided by the present invention.

### ADDITIONAL ASPECTS OF THE INVENTION

A1. An orally administered pharmaceutical composition comprising a first water-swellable gel-forming layer and a second water-swellable gel-forming layer in the outermost layer, wherein the outer edge of the first water-swellable gel forming layer is bonded to the outer edge of the second water-swellable gel-forming layer so as to enclose a drug inside the orally administered pharmaceutical composition.
A2. The orally administered pharmaceutical composition according to A1, comprising a drug-containing layer provided between the first water-swellable gel-forming layer and the second water-swellable gel-forming layer, wherein the outer edge of the first water-swellable gel forming layer is bonded to the outer edge of the second water-swellable gel-forming layer so as to enclose the drug-containing layer inside the orally administered pharmaceutical composition.
A3. The orally administered pharmaceutical composition according to A1 or A2, wherein the outer edge of the first water-swellable gel-forming layer and the outer edge of the second water-swellable gel-forming layer are directly bonded together.
A4. The orally administered pharmaceutical composition according to A1 or A2, wherein the outer edge of the first water-swellable gel-forming layer and the outer edge of the second water-swellable gel-forming layer are bonded together via an adhesive layer.

## Claims

1. An orally administered pharmaceutical composition comprising a first water-swellable gel-forming layer and a second water-swellable gel-forming layer in the outermost layer,
wherein the outer edge of the first water-swellable gel-forming layer and the outer edge of the second water-swellable gel-forming layer are bonded together so as to enclose a tablet inside the orally administered pharmaceutical composition.

2. The orally administered pharmaceutical composition according to claim 1,
wherein the outer edge of the first water-swellable gel-forming layer and the outer edge of the second water-swellable gel-forming layer are bonded together by heat fusion via an adhesive layer containing a heat-fusable adhesive.

3. The orally administered pharmaceutical composition according to claim 2,
wherein the adhesive layer is layered on the bottom surface of the first water-swellable gel-forming layer and the adhesive layer is layered on the top surface of the second water-swellable gel-forming layer.

4. The orally administered pharmaceutical composition according to claim 2 or 3,
wherein the heat-fusable adhesive is a homopolyer of polyvinylpyrrolidone.

5. A method of producing a orally administered pharmaceutical composition, the method comprising:
applying a suspension containing a water-swellable gel-forming agent and a film-forming agent to the upper surface of a support base, drying to form water-swellable gel-forming layer; applying a suspension containing a heat-fusable adhesive to the upper surface of the water-swellable gel-forming layer and drying to produce a first layered body comprising a first water-swellable gel-forming layer and an adhesive layer containing a heat-fusable adhesive layered in that order on a first support base;
applying a suspension containing a water-swellable gel-forming agent and a film-forming agent to the upper surface of a support base, drying to form water-swellable gel-forming layer; applying a suspension containing a heat-fusable adhesive to the upper surface of the water-swellable gel-forming layer and drying to produce a second layered body comprising a second water-swellable gel-forming layer and an adhesive layer containing a heat-fusable adhesive layered successively on a second support base; forming a drug-containing layer on the upper surface of the adhesive layer so that the outer edges of the upper surface of the adhesive layer remain exposed; and
a step of bonding the outer edge of the first water-swellable gel-forming layer and the outer edge of the second water-swellable gel-forming layer together by heat fusion via the adhesive layer layered on the first water-swellable gel-forming layer and the adhesive layer layered on the second water-swellable gel-forming layer, so as to enclose the drug-containing layer inside the orally administered pharmaceutical composition;
wherein the drug-containing layer is a tablet.
